Europäisches Patentamt

(19) European Patent Office

Office européen des brevets

(11) Numéro de publication: **0 341 143**
**A1**

(12) **DEMANDE DE BREVET EUROPEEN**

(21) Numéro de dépôt: **89401219.4**

(22) Date de dépôt: **28.04.89**

(51) Int. Cl.⁴: **A 61 B 6/03**

(30) Priorité: **06.05.88 FR 8806138**

(43) Date de publication de la demande:
**08.11.89 Bulletin 89/45**

(84) Etats contractants désignés: **DE ES GB IT NL**

(71) Demandeur: **GENERAL ELECTRIC CGR SA**
**100 rue Camille Desmoulins**
**F-92130 Issy les Moulineaux (FR)**

(72) Inventeur: **Trotel, Jacques**
**CABINET BALLOT-SCHMIT 84, Avenue Kléber**
**F-75116 Paris (FR)**

(74) Mandataire: **Ballot, Paul Denis Jacques**
**Cabinet Ballot-Schmit 84, avenue Kléber**
**F-75116 Paris (FR)**

(54) Dispositif et procédé de tomographie à grande cadence d'acquisition.

(57) Pendant l'examen d'un corps (16) par balayage tomographique l'acquisition de données de reconstruction d'image peut être accélérée par la commande des mouvements d'un ensemble (1) tube à rayons X (2)-multidétecteur (4) de sorte qu'il fasse en alternance des translations rapides et des rotations pas-à-pas. L'avantage d'un multidétecteur du type multirangées (19 à 23) se manifeste à vitesse égale, par l'amérioration de la qualité des images produites, ou, à qualité d'image égale, par la réduction du temps d'acquisition. En outre, ce mode de fonctionnement est particulièrement adapté à la surveillance d'actions de radiothérapie dans lesquelles le praticien, au cours de l'acquisition des images, manifeste le souhait de pouvoir intervenir sur le patient.

FIG_2

**Description**

## DISPOSITIF ET PROCEDE DE TOMOGRAPHIE A GRANDE CADENCE D'ACQUISITION

La présente invention a pour objet un dispositif et un procédé de tomographie à grande cadence d'acquisition, utilisable en particulier dans le domaine médical.

Elle concerne d'une manière générale les tomographes dits à image reconstruite par traitement numérique des informations acquises. Elle a pour principal but de réduire les temps d'attente, surtout ceux supportés par des patients humains, nécessaires à l'acquisition d'images en mode multicoupe, ou à trois dimensions. Elle est aussi destinée à équiper d'une manière complémentaire des appareils de simulation de radiothérapie. Pour de tels appareils la contrainte essentielle consiste à permettre au praticien d'accéder à tout moment sur n'importe quel organe d'un patient, même pendant la phase d'acquisition des tomographies.

On connaît les tomographes à images reconstruites, dans lesquels un ensemble tube à rayons X-multidétecteur, maintenus fixement l'un par rapport à l'autre sensiblement dans un même plan, permet d'acquérir des informations relatives à l'image d'une coupe, selon ce plan, d'un objet interposé entre ce tube à rayons X et ce multi-détecteur. En effet pour chaque orientation d'une direction médiane du rayonnement du tube à rayons X par rapport à l'objet, on peut acquérir un ensemble d'informations représentatives d'une vue d'une tranche de cet objet selon cette orientation. En réitérant l'expérience pour différents orientations, on peut acquérir un ensemble de vues à partir desquelles, sous certaines conditions, on peut au moyen d'un algorithme de traitement appelé communément algorithme de rétroprojection, reconstruire l'image de la coupe de l'objet selon ce plan.

La durée totale d'acquisition des informations qui permettent de reconstruire cette image dépend de la vitesse de rotation de l'ensemble tube à rayons X-multidétecteur, de la puissance du tube à rayons X et de la sensibilité du détecteur. Intuitivement on comprend que plus un tube à rayons X sera puissant, plus il émettra de rayons X d'une dureté donnée (et non pas des rayons X plus durs), plus il sera facile de détecter avec le multidétecteur le résidu de ces rayonnements après passage à travers des régions de l'objet même les plus opaques à ce rayonnement. Il sera alors possible de faire tourner l'ensemble tube-détecteur plus vite pour acquérir l'image plus rapidement. Dans le même esprit, plus le détecteur est sensible, plus son rendement quantique est meilleur, plus la vitesse d'acquisition pourra être également augmentée. Ceci étant, pour une technologie donnée de l'ensemble tube à rayons X -multidétecteur, la vitesse de rotation de l'ensemble est un facteur déterminant de la qualité de l'image reconstruite. En effet, plus la vitesse est lente, plus il est possible de laisser les multidétecteurs sous une influence longue du tube du rayons X à chaque vue, et plus les informations délivrées par chacune des cellules de ce multidétecteur seront précises et représentatives. A l'opposé, plus on va vite, et plus l'image se dégrade quant à sa précision ou sa résolution.

Les machines de tomographie à image reconstruite actuellement disponibles sont principalement des machines dites de troisième génération. Dans ces machines, l'ensemble tube à rayons X-multidétecteur, bien que tournant, est relié par des câbles aux circuits d'alimentation et de réception de la machine. Une acquisition nécessite alors l'accélération au démarrage de l'ensemble tube à rayons X-multidétecteur, la stabilisation en vitesse de cet ensemble, l'acquisition proprement dite des informations à une vitesse stabilisée, et le ralentissement puis l'arrêt de l'ensemble (avant arrachement des connexions des câbles). Tout ce déplacement en rotation nécessite à l'ensemble d'effectuer environ deux tours sur lui-même. Pour une autre acquisition, pour l'image d'une coupe adjacente à une coupe précédemment acquise, cet ensemble d'opérations est recommencé en faisant tourner la machine dans l'autre sens et ainsi de suite. De ce mode de fonctionnement, il résulte que la durée de l'acquisition en pratique, n'est pas liée qu'aux choix évoqués précédemment de qualité d'image, mais est surtout liée à ce type de mise en marche. En pratique, la durée proprement dite d'acquisition varie entre une demi seconde pour les images dont la précision requise doit être faible, jusqu'à dix secondes pour les images finement résolues. Dans les deux cas, l'acquisition des informations relatives à une coupe, compte tenu de l'accélération et du ralentissement, est de l'ordre d'une dizaine de secondes.

Les multidétecteurs les plus connus sont des multidétecteurs à gaz comportant un ensemble de cellules remplis de gaz (généralement du Xénon). Chaque cellule du multidétecteur comporte deux plaques métalliques, polarisées électriquement à des tensions inverses l'une de l'autre. Sous l'effet du rayonnement X qui traverse l'objet, ce gaz s'ionise et autorise le passage d'un courant de fuite entre les deux plaques polarisées à des hautes tensions opposées. La mesure de ces courants de fuite permet de détecter les informations nécessaires pour reconstruire l'image. Un multidétecteur comporte donc une multitude de cellules du même type, alignées les unes derrière les autres en une rangée dont la direction principale est contenue dans le plan de coupe de l'ensemble tube à rayons X-multidétecteur. De telles cellules ont une largeur, mesurée perpendiculairement à ce plan de coupe, de l'ordre de 1,5 cm. De manière à acquérir des images les plus fines possibles, on utilise un colimateur ayant pour objet de réduire le rayonnement omnidirectionnel du tube à un rayonnement en un faisceau plat en éventail, illuminant tout le détecteur, et dont la largeur correspond au pas de résolution en épaisseur de la coupe à reconstruire. En pratique, cette épaisseur peut varier, d'une machine à une autre, de 1 millimètre à 10 millimètres. Lorsque cette épaisseur vaut 10 millimètres, le débit des rayons X sur le multidétecteur peut atteindre plus rapidement un niveau sensible, même pour les régions les plus opaques de l'objet, de telle façon que de telles images qui sont les moins bonnes en résolution sont cependant les images acquises le plus rapidement.

Lorsqu'on veut réaliser une représentation en trois dimensions de l'objet étudié, on réalise des images de plusieurs sections adjacentes de cet objet. Ceci est obtenu en déplaçant l'objet, après la réalisation de chaque coupe, selon un axe perpendiculaire au plan de coupe d'une longueur égale à l'épaisseur du faisceau. Dans un exemple si on désire acquérir une connaissance à trois dimensions d'un objet, sur 25 centimètres de longueur de cet objet, et avec une résolution d'un millimètre dans le sens de cette longueur, il y a lieu de réaliser 250 coupes adjacentes. Pour une résolution moyenne la durée de rotation (accélération, stabilisation, acquisition, ralentissement) y compris les translations latérales de 1 millimètre entre chaque image, peut être de l'ordre de quatre secondes. La durée totale d'acquisition est alors d'environ 1000 secondes.

L'ensemble tube-détecteur ainsi décrit, compte tenu du nombre de tours à effectuer à chaque acquisition, doit être contenu dans un capotage circulaire fixe empêchant des opérateurs qui interviennent sur cette machine d'être écharpés. L'acquisition des différentes coupes se fait par déplacement relatif de ce capotage par rapport à un lit d'examen sur lequel est placé l'objet, le patient dans le domaine médical. En pratique, le capotage est fixé au sol, le lit se déplace en translation longitudinale dans la machine. Un tel capotage circulaire ne permet pas à un praticien d'avoir accès à tout moment à tous les organes d'un patient. Par exemple, si ce patient subit un examen dans la région du coeur, sa tête se trouve d'un côté de la machine alors que son bas du tronc et ses membres inférieurs sont placés de l'autre côté. Cet inconvénient est particulièrement gênant si le tomographe est utilisé dans un appareil dénommé "simulateur de radiothérapie".

Le but d'un tel appareil est de fournir des informations sur l'anatomie interne et externe d'un patient pour déterminer à l'avance les caractéristiques des faisceaux qui seront employés lorsque ce patient sera placé ultérieurement sur un appareil de radiothérapie, de telle façon que le rayonnement de radiothérapie délivre la dose voulue aux régions à traiter et la dose la plus faible possible aux autres régions de ce patient.

Pour l'examen sur le simulateur de radiothérapie le patient doit être placé dans la position exacte qu'il aura sur l'appareil de radiothérapie. Ces positions sont codifiées pour donner le maximum d'efficacité au traitement. Elles sont souvent incompatibles avec la géométrie des appareils classiques de tomographie, d'autant plus que l'opérateur doit aider le patient à prendre ces positions. D'autre part, pendant l'examen sur le simulateur, l'opérateur doit tracer sur la peau du patient des repères qui permettront de placer des faisceaux de thérapie. Ces considérations imposent que le voisinage du patient soit facilement accessible pendant l'examen sur le simulateur.

L'enlèvement du capotage, au demeurant volumineux, ne résout de plus pas le problème, car en l'absence de ce capotage, les opérateurs, et le patient prennent des risques d'être agrippés par la machine. Pour éviter ces situations à risques, des contraintes de limitation de vitesse ont été imposées par les autorités légales. Ces contraintes ont pour objet de limiter les vitesses des déplacements non capotés de telle façon que l'ensemble source-multidétecteur ne puisse pas franchir un tour sur lui-même en moins de 60 secondes. Dans ces conditions, l'acquisition de tomographies en vue de reconstruire une représentation à trois dimensions d'un objet serait inconcevable.

Dans l'invention, on remédie à ces inconvénients en réalisant que lorsque le nombre de coupes exigées est important (en particulier lorsque la résolution longitudinale exigée est fine) l'utilisation préconisée jusqu'ici des tomographes à images reconstruites n'est pas la meilleure. Plutôt que d'acquérir alors chaque image par un ensemble de vues obtenues par une rotation de l'ensemble autour de l'objet, et de faire suivre chaque acquisition d'une translation élémentaire pour acquérir une image suivante, on a eu l'idée, au contraire, de provoquer un balayage longitudinal de l'objet par l'ensemble tube-détecteur, et de faire suivre chaque translation par une rotation d'un pas de cet ensemble. A chaque pas de rotation, l'exploration de l'objet par un balayage longitudinal est entreprise. De préférence, dans un perfectionnement, le balayage est aval (va de la tête vers les pieds) puis est amont (va des pieds vers la tête) au pas de rotation suivant et ainsi de suite.

Les ordres de grandeurs comparatifs sont les suivants : dans l'état de la technique, 250 images comportant 1000 vues chacune et durant chacune 4 secondes, y compris la translation entre images, conduisaient à une durée totale d'acquisition de 1000 secondes. Avec la technique de l'invention, on procédera en correspondance à 1000 translations suivies chacune d'un déplacement en rotation d'un pas de l'ensemble tube à rayons X-multidétecteur. Il est à remarquer que ces déplacements pas à pas en rotation sont de très faible amplitude : ils peuvent donc être très rapides. En effet ils sont de l'ordre de 18 minutes d'angle (à un degré d'angle selon la qualité de l'examen). Leur exécution rapide ne peut faire courir de risque aux opérateurs proche de la machine. De même les translations sur environ 30 centimètres (25 centimètres d'exploration et 2 fois 2.5 centimètres d'accélération et de ralentissement de part et d'autre) peuvent être également rapides. On peut aboutir dans ces conditions à une même durée totale d'acquisition de 1000 secondes. Cependant dans ce cas, la solution selon l'invention présente l'avantage que les accélérations et ralentissements de l'ensemble étant relativement moins longs, la durée relative d'utilisation du multidétecteur à des fins de détection peut être plus longue. En définitive, la sensibilité de la machine en est augmentée. Par exemple, si la durée de balayage longitudinal est de l'ordre de 0.75 secondes pour 25 centimètres, la durée d'irradiation du multidétecteur, relative à une tranche d'épaisseur 1 millimètre, est de l'ordre de 3 millisecondes (0,750/250). En comparaison avec les machines de troisième génération, soit cette durée d'utilisation est moins grande pour que la durée totale d'acquisition soit la même, soit cette durée intrinsèque d'utilisation est la même, mais la durée totale d'acquisition est alors plus longue.

Dans un perfectionnement en outre, ces résultats encore en partie comparables sont considérablement augmentés dans l'invention si le multidétecteur est du type multirangées. En effet, on peut choisir un multidétecteur du type de celui décrit dans le brevet européen n°0 051 350 déposé le 28 juillet 1981. Ce multidétecteur comporte un réseau, à deux dimensions, de cellules détectrices. Dans ce cas le traitement est de type discret. Pendant la translation, les charges électrique représentatives des informations des cellules d'une des rangées du multidétecteur multirangées sont transférées dans des cellules adjacentes d'une rangée adjacente, dans un sens inverse au mouvement de la translation. Le contenu d'information des cellules de la dernière rangée rend compte de l'effet d'intégration résultant de ces transferts. Il est exploité comme représentatif de la vue.

Pour simplifier on peut indiquer que, sous une tranche donnée de l'objet, passent dans l'ordre, du fait du déplacement, des rangées de cellules numérotées 1 à n (n valant par exemple 5). A chaque fois qu'une autre rangée est placée sous la tranche, le contenu d'information des cellules de la rangée précédente est transféré dans les cellules de cette rangée. Ainsi de suite jusqu'à ce que la rangée numéro n des cellules soit sous la tranche. A l'issue de l'irradiation de cette rangée sous cette tranche, le signal est prélevé par échantillonnage. Ce procédé a l'avantage de soumettre en définitive la tranche examinée à une irradiation n fois plus longue. Il en résulte immédiatement qu'à qualité de résolution égale, l'image peut alors être acquise n fois plus vite ; ou bien à vitesse d'acquisition égale, sa résolution peut être n fois meilleure.

Le dispositif selon l'invention souscrit par ailleurs totalement aux exigences légales évoquées ci-dessus. En effet à chaque rotation d'un angle faible, de l'ordre d'une fraction de degrés, il n'y a pas lieu de prendre trop de précautions. Le mouvement peut être donc très rapide puisque par ailleurs il a un débattement très faible. En outre, les translations, de l'ordre de 30 centimètres, peuvent se faire à l'intérieur d'un capot. Sur 30 centimètres en effet, un opérateur pourrait être blessé s'il était agrippé par la machine. Mais, du fait qu'avec l'invention on n'exécute plus de tours entiers comme dans les machines de troisième génération, il n'est plus nécessaire de créer une structure de type circulaire pour ce capot. Une structure en forme générale d'arc de cercle suffit. Par l'intérieur de l'arc du cercle il devient alors possible d'accéder à tous les organes du patient.

L'invention a donc pour objet un dispositif de tomographie comportant
- un ensemble tube à rayons X-multidétecteur muni d'un tube à rayons X agencé pour rayonner en direction d'un multidétecteur avec lequel il est mécaniquement associé.
- des moyens de rotation pour faire tourner cet ensemble autour d'un axe de rotation passant transversalement entre le tube à rayons X et le multidétecteur, cette rotation correspondant à un angle minimum d'exploration nécessaire à une reconstruction d'un objet interposé entre le tube et le multidétecteur,
- des moyens de translation pour déplacer au moins le multidétecteur le long de cet axe de rotation, la longueur de cette translation correspondant à la longueur d'une région à examiner de l'objet,
- et des moyens de traiter des signaux délivrés par le multidétecteur, ces signaux résultant d'émissions du tube à rayons X, en vue de produire des images de l'objet interposé,
caractérisé en ce qu'il comporte en outre
- des moyens pour faire tourner l'ensemble d'un angle égal à cet angle minimum, de telle façon qu'une vitesse moyenne tangentielle du multidétecteur soit moins rapide que sa vitesse de translation et donc que cette rotation se produise pendant une durée totale plus longue que la durée nécessaire pour que cet ensemble ne se déplace en translation au moins une fois le long de toute cette longueur à examiner,
- et des moyens pour faire émettre le tube à rayons X au cours du déplacement en translation.

L'invention sera mieux comprise à la lecture de la description qui suit et à l'examen des figures qui l'accompagnent. Celles-ci ne sont données qu'à titre indicatif et nullement limitatif de l'invention. Les figures montrent :
- figure 1 : un dispositif de tomographie selon l'invention ;
- figure 2 : un détail du dispositif de la figure 1 montrant des particularités de fonctionnement d'un multidétecteur multirangées ;
- figures 3a et 3b : une comparaison des modes d'exploration, respectivement dans l'état de la technique et selon l'invention ;
- figures 4a à 4c : des diagrammes temporels de signaux intervenant dans la commande des moyens moteurs et dans le pilotage des moyens de détection du dispositif selon l'invention ;
- figure 5 : une particularité du mode d'acquisition et de reconstruction selon l'invention ;
- figures 6a et 6b : des indications permettant la modification des algorithmes de reconstruction en fonction d'un mode préconisé d'acquisition.
- figure 7 : une variante des diagrammes temporels des figures 4a à 4c.

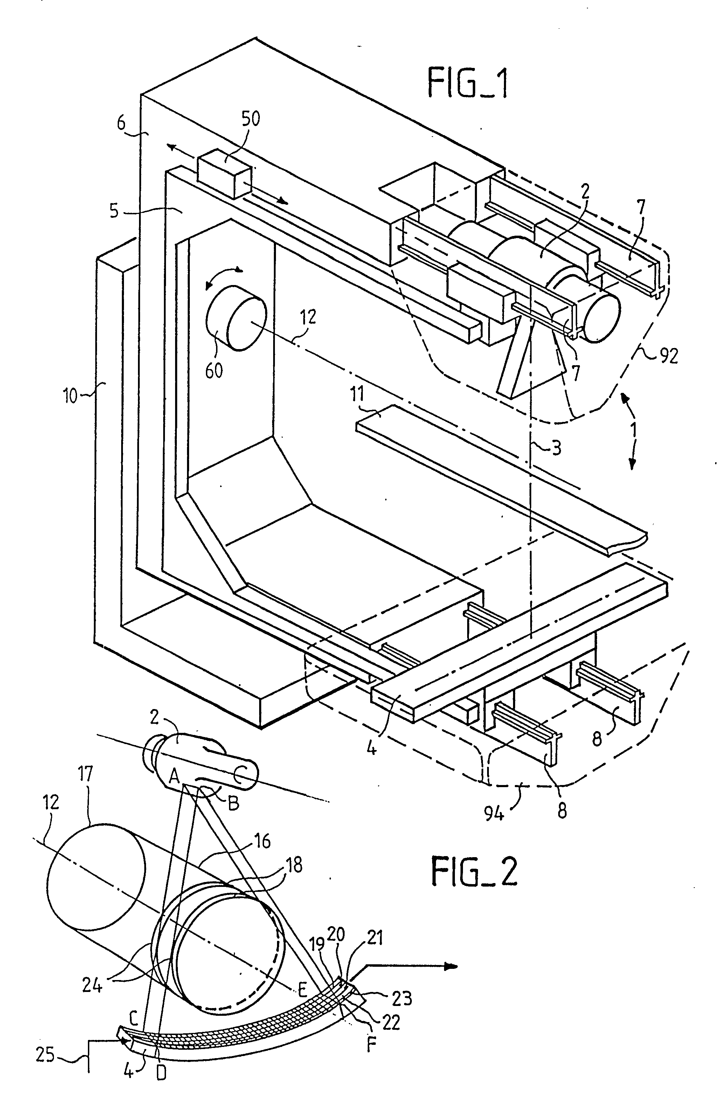

La figure 1 représente une dispositif de tomographie conforme à l'invention. Ce dispositif comporte un ensemble 1 tube à rayons X-multidétecteur. Dans cet ensemble un tube 2 à rayons X rayonne un rayonnement X 3 en direction d'un multidétecteur 4 avec lequel il est mécaniquement associé. Par exemple, le tube 3 et le multidétecteur 4 sont schématiquement associés par une structure 5 de liaison, de manière à ce qu'ils subissent ensemble des déplacements : le rayonnement X étant toujours orienté correctement vers le multidétecteur 4. L'ensemble 1 est monté dans un arceau 6 en forme générale d'arc de cercle. Cet arceau 6 est relié à un socle 10 par un palier permettant une rotation de cet arceau 6 par rapport à un axe 12 perpendiculaire au faisceau de rayons X3. L'ensemble 1 et la structure 5 sont montés sur l'arceau 6 par l'intermédiaire de glissières 7 et 8 permettant de donner à l'ensemble 1 un mouvement de translation parallèle à l'axe 12. Des moteurs 50 et 60 donnent respectivement à la structure 5 un mouvement de translation parallèle à l'axe 12, et à l'arceau 6 un mouvement de rotation selon l'axe 12. Un patient, ou un objet à examiner, sont disposés sur un plateau 11 sensiblement parallèlement à l'axe 12 et sont destinés à venir s'interposer sur le trajet du rayonnement X 3. Ce mécanisme sert de moyen pour faire tourner l'ensemble tube-multidétecteur autour de l'axe 12 orienté parallèlement au plateau 11. Le tube 2 et le multidétecteur 4 sont respectivement contenus dans des capotages 92 et 94 indiqués en tirets. Les capotages 92 et 94 sont fixés à l'arceau 6. Ils permettent le débattement utile en translation, dans leur volume intérieur, du tube et du multidétecteur.

Avec les moyens de rotation le dispositif de l'invention comporte aussi, classiquement, des moyens de translation pour déplacer cet ensemble relativement au panneau 11. En pratique le panneau 11 peut être déplacé longitudinalement et verticalement. Des circuits non représentés, par exemple placés dans la colonne-socle 10, permettent de prélever et de traiter le signal délivré par le multidétecteur 4 en vue de produire et de visualiser des images de coupes d'objets interposés, sur le plateau 11, sur le trajet du rayonnement 3.

La caractéristique essentielle de l'invention réside dans son utilisation. Pour obtenir un ensemble de tomographies, on déplace l'ensemble 1, y compris ses capotages 92 et 94, pas-à pas en rotation, et à chaque pas, alors que cet ensemble 1 est maintenu fixe en rotation, on le translate selon l'axe 12 passant par son centre de rotation. De cette manière on balaye toute une portion correspondante à examiner de l'objet. Cette translation ne se fait pas en déplaçant ni le plateau 12, ni les capotages 92 et 94. En effet, comme on l'a indiqué précédemment, ce déplacement est assez important, il doit être de l'ordre de 30 centimètres. Comme par ailleurs il doit être rapide pour permettre l'acquisition rapide de toutes les images, ce déplacement de l'ensemble 1 se fait de préférence à l'intérieur des capotages 92 et 94 fixes par rapport à l'arceau 6. Dans ce but, la structure 5 de liaison par exemple de type treillis, peut être entraînée en translation par des moteurs tels que 50, dans un sens, puis dans un autre à un pas de rotation suivant. Les moteurs tel que 50 peuvent être de tous types, électriques, pneumatiques. Ils peuvent même être remplacés par des moteurs indépendants directement placés sur le tube 2 et sur le multidétecteur 4, et asservis en position l'un par rapport à l'autre pour assurer en permanence une bonne irradiation du multidétecteur 4 par le rayonnement X 3. Dans ce dernier cas la structure 5 peut être enlevée.

Compte tenu de problèmes d'inertie qui peuvent être posés du fait de la fragilité de la structure en treillis 5, ou tout simplement du fait des poids du multidétecteur 4 et du tube 2, il peut se poser des problèmes de défaut d'irradiation. Cependant, dans l'invention, pour remédier à ce problème et surtout pour permettre une augmentation, à qualité constante, de la cadence d'acquisition, on réalise un multidétecteur du type décrit dans le brevet européen précité ou encore, dans un mode non discret, du type de celui décrit par FRANCK A. di BIANCA dans un article intitulé "KINESTATIC charge détection" et publié dans la revue MED.PHYS. 12(3), de mai-juin 1985, pages 339 à 343. Dans cet article, il est prévu que l'intégration des signaux est obtenue par une dérive d'ions dans un détecteur à gaz. Cette dérive est en sens inverse au mouvement de balayage. Le mode d'acquisition ainsi préconisé avec intégration rend la résolution longitudinale des acquisitions dépendante, non plus de la finesse d'un colimateur présent entre le tube à rayons X et le multidétecteur, mais du mode d'échantillonnage et d'intégration des signaux délivrés par un tel multidétecteur. Ce multidétecteur devient assimilable à un multidétecteur multirangées. Les rangées sont parallèles les unes aux autres, sensiblement perpendiculairement au sens de déplacement. Il en résulte que des distorsions d'acquisition, dues à des charges inertielles différentes au moment de la mise en translation peuvent être ignorées si on ne se préoccupe que du repérage précis de la position du multidétecteur 4 dans le capotage 94 au moment des échantillonnages. On peut alors choisir un colimateur large tout en n'altérant pas la finesse de la résolution longitudinale.

On remarque sur la figure 1 que les formes de l'arceau 6 et des capotages 92 et 94 permettent d'accéder à tout moment à tous les organes du patient sur le plateau 11. Cette caractéristique est importante pour l'application à un simulateur de radiothérapie comme il est indiqué ci-dessus. Cette caractéristique s'adapte plus généralement aux examens où le patient ne peut pas prendre une position compatible avec un appareil classique, et aux examens nécessitant l'assistance d'un opérateur ou la présence d'accessoires encombrants mais indispensables, comme dans certains examens de traumatologie.

La figure 2 permet d'expliquer le fonctionnement du multidétecteur multirangées. Elle représente schématiquement le déplacement du tube 2 et du multidétecteur 4 par rapport à un corps 16 globalement placé selon l'axe 12 de la machine. On suppose d'abord que le tube 2 émet un rayonnement X 3 en éventail, selon le contour ABCDEF, de type parallèle dans une tranche 18. On peut admettre, pendant le déplacement de l'ensemble 1 vers la partie arrière 17 de l'objet 16, que chaque tranche 18 irradiée de cet objet, et dont le signal d'absorption radiologique est prélevé par le multidétecteur multirangées 4, peut être divisée en un nombre n de tranches élémentaires correspondant chacune à une des rangées du multidétecteur multirangées. Par exemple sur la figure 2, le multidétecteur multirangées 4 comporte cinq rangées cotées 19 à 23. A un instant donné le multidétecteur est placé sous la tranche 18. A un instant suivant, lorsque la tranche irradiée 18 devient la tranche 24 qui interpénètre en partie la tranche 18, une bonne partie de cette tranche 18 continue à être irradiée. Cependant la partie de cette tranche 18 dont le signal d'absorption radiologique aboutissait sur la rangée 19 du multidétecteur multirangées aboutit maintenant sur la rangée 20, tandis que le signal d'absorption radiologique qui aboutissait sur la rangée 20 aboutit maintenant sur la rangée 21, et ainsi de suite.

Avant que le tube 2 et le multidétecteur 4 ne se déplacent un peu plus vers l'arrière 17 du corps 16 il importe de prélever le signal disponible dans la rangée 23. Un ordre de cadencement 25 appliqué au multidétecteur multirangées 4 permet de transférer, à chaque impulsion de cadence, les charges électriques des cellules de chaque rangée dans les cellules correspondantes d'une rangée adjacente, mais adjacente d'un sens opposé au sens du déplacement. L'ordre de cadencement 25 sert également à permettre l'échantillonnage du signal délivré par la dernière rangée 23 du multidétecteur 4. Le transfert des charges électriques d'une cellule à une autre, dans un cas discret, ou le long d'une cellule à gaz par dérive continue d'ions comme décrit dans l'article précité, est tout à fait transposable dans l'invention. Le cadencement permet d'organiser ce transfert ainsi que le prélèvement, l'échantillonnage, dans la dernière rangée des informations utiles. Dans le cas discret, à chaque impulsion de cadence, le contenu d'information d'une cellule vient remplacer le contenu de la cellule adjacente. Les cellules de la rangée du front d'attaque de balayage sont remises à zéro à chaque impulsion.

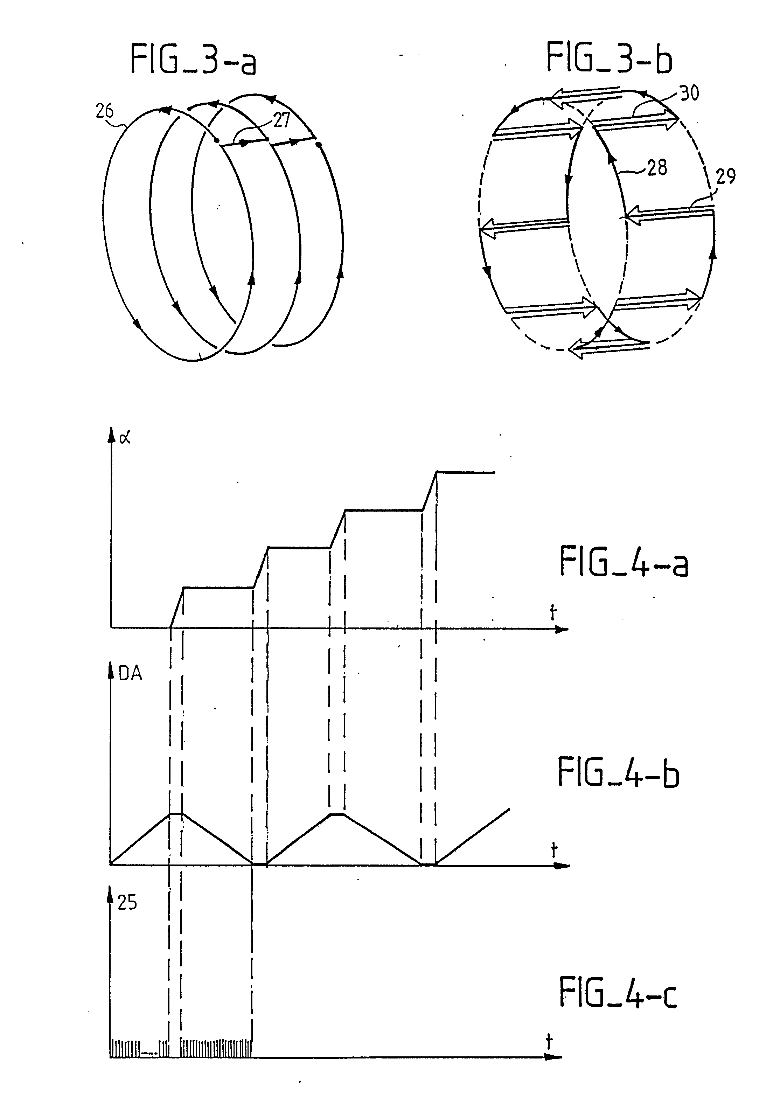

Les figures 3a et 3b montrent d'une manière comparée les modes d'acquisition dans l'état de la technique et dans l'invention respectivement. Dans l'état de la technique l'acquisition d'une image se produisait lorsque l'ensemble tube à rayons X-multidétecteur effectuait des tours sur lui-même tels que les tours 26. Après chaque tour, une légère translation 27 permettait de placer l'ensemble à l'aplomb d'une autre coupe du corps à examiner. Dans l'invention (figure 3b), l'arceau 6 est soumis à des légères rotations pas à pas 28 (montrées ici d'une manière exagérée), et, entre chaque pas, à l'intérieur des capotages 92 et 94, l'ensemble tube à rayons X 2-multidétecteur 4 est soumis à des translations telles que 29.

D'une manière préférée, avec un multidétecteur multirangées à transfert de charge et bidirectionnel (c'est à dire pouvant transférer les charges dans un sens et, par modifications rapides, dans un sens opposé), après chaque rotation d'un pas 28 le déplacement en translation est de sens opposé, à l'intérieur des capotages 92 et 94, au sens 29 d'un déplacement précédent. Les figures 4a à 4c montrent respectivement, au cours de temps, les pas de rotation $\alpha$ des capotages 92 et 94, les déplacements alternatifs DA de l'ensemble 1 dans les capotages, ainsi que le cadencement des ordres 25. Pendant la durée d'une acquisition, pendant une première période les capotages sont fixes ($\alpha$ ne varie pas), le déplacement est linéaire (DA suit une rampe régulière), et le nombre des ordres 25 correspond sensiblement au nombre des tranches à acquérir pendant toute la durée du balayage. Ce nombre correspond également à la résolution longitudinale à obtenir. Par exemple sur 250 millimètres, avec une résolution de 1 millimètre, il y a 250 impulsions de cadencement 25.

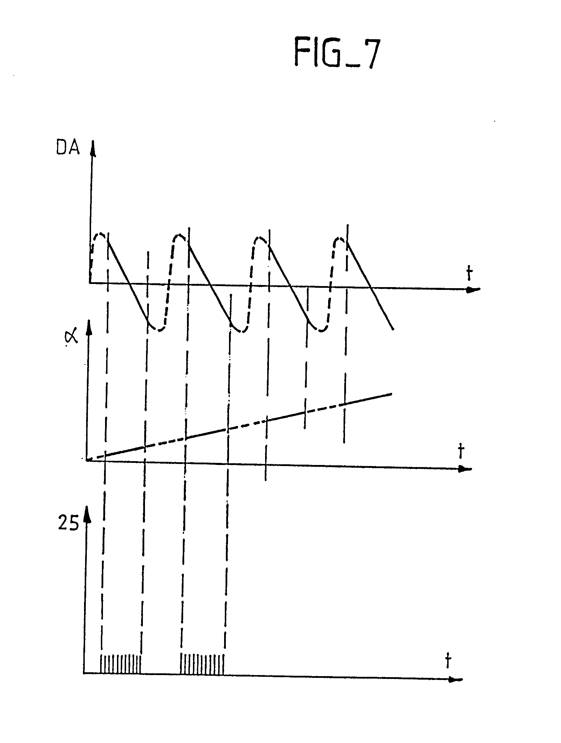

Dans le cas où le détecteur n'est pas bidirectionnel, le mode de réalisation préféré correspond à la figure 7, c'est à dire à une rotation continue et à un mouvement de la translation asymétrique. Ce déplacement asymétrique comporte un déplacement lent pendant l'acquisition dans un sens toléré par le détecteur, et un déplacement de retour rapide dans un sens ou le détecteur est inactif.

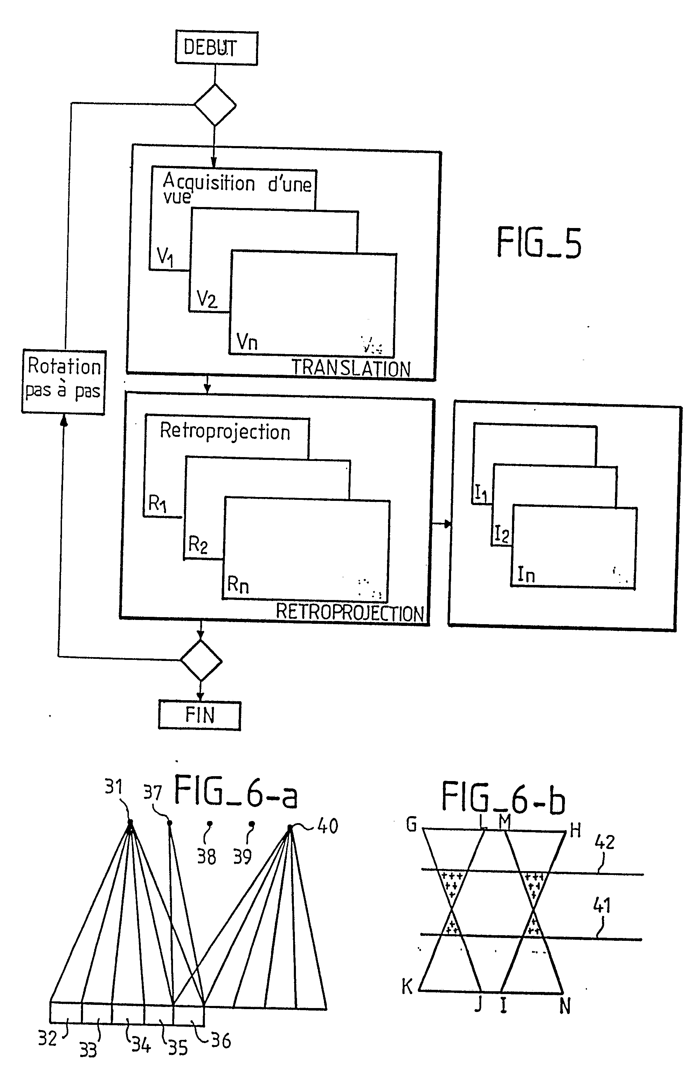

La figure 5 montre une particularité du mode d'acquisition et de reconstruction des images obtenues selon l'invention. En effet, dans l'état de la technique l'acquisition d'une vue $V_1$ est normalement classiquement suivie d'une rétroprojection $R_1$, permettant de définir en une première phase une image $I_1$ correspondante. Lorsque le tomographe tourne sur lui-même, il acquiert ensuite des vues $V'_1$ puis $V''_1$ ... qui sont transformées par des opérations de rétroprojection $R'_1$ puis $R''_1$... en vue de compléter la définition de l'image $I_1$. Lorsque l'image $I_1$ est complètement définie, le tomographe de l'état de la technique est translaté (27) à l'aplomb de la tranche suivante et le processus est recommencé.

Dans l'invention, l'algorithme de rétroprojection utilisable sera le même. Tout simplement entre l'acquisition d'une vue $V_1$ et l'acquisition de la vue $V'_1$ suivante correspondant à une même coupe dans l'objet, on aura, par le jeu de la translation de balayage 29, acquis des coupes $V_2...V_N$ qui sont respectivement soumises à des rétroprojections $R_2...R_N$ en vue de définir en une première passe les autres images $I_2...I_N$ (dans l'exemple N vaut 250). La conséquence de cette situation est que sur le plan pratique, les capacités mémoire et les algorithmes de traitement sont les mêmes. Seule l'organisation de l'acquisition change( en correspondance. Dans le cas décrit où, d'une translation 29 à une autre 30, le sens d'exploration de l'objet 16 est inversé, il importe, sachant qu'on acquiert alors la vue $V'_N$ avant la vue $V'_1$, d'effectuer dans l'ordre convenable les rétroprojections $R_N ...R_1$ en vue de compléter la définition en une passe suivante des images $I_N ...I_1$.

Sur la figure 2 on a représenté un rayonnement de type idéal dans lequel la source de rayonnement X n'est pas ponctuelle mais est étendue sur une distance AB et dans lequel le rayonnement est de type parallèle. La réalité est différente, et la source de rayons X est ponctuelle. Sur la figure 6a on a représenté, au cours d'une translation, la partie réellement irradiée et prise en considération du corps examiné. On a représenté une première position 31, du foyer de rayons X, ainsi qu'un alignement de cellules 32 à 36 appartenant par exemple respectivement aux rangées 19 à 23 du multidétecteur multirangées de la figure 2. A chaque fois que la source de rayons X se déplace vers une position adjacente, par exemple les positions 37 à 40, les contenus électriques des cellules 36 à 32 sont décalés en sens inverse : c'est à dire de la cellule 36 vers la cellule 32.

On observe que quand le tube à rayons X se trouve en 31, la zone de l'objet irradié, dont la mesure d'irradiation est détectée par la cellule 36, est contenue dans le triangle issu de la source 31 et aboutissant à cette cellule 36. Lorsque la source s'est déplacée en 37 la cellule 36 s'est déplacée vers la droite, et la cellule 35 est venue prendre la place de la cellule 36 sous le même aplomb du corps examiné. Cette cellule 35 reçoit alors un signal d'absorption radiologique correspondant à ce qui s'est passé dans le triangle dont le sommet est en 37 et dont la base est également en 36. En outre, du fait du transfert, le contenu des charges électriques de la cellule 36 a été transféré à la cellule 35, provoquant le phénomène d'intégration propice à l'augmentation de sensibilité de l'invention. Ainsi de suite jusqu'à ce que le tube occupe la position 40 et que la cellule 32 soit placée à l'endroit où, sur le dessin, se trouve actuellement la cellule 36.

Il en résulte que dans ces conditions, la partie étudiée de l'objet n'est pas parfaitement une tranche plane comme le suggérait la tranche 18 de la figure 2, mais est au contraire une tranche de section trapézoidale dont la grande base est égale à la largeur du multidétecteur multirangées et dont la petite base est égale à la largeur d'une des rangées du multidétecteur multirangées. On en déduit qu'on ne peut pas utiliser dans ces conditions un détecteur multirangées avec trop de rangées ou un détecteur trop large. Le nombre de rangées égal à 5 semble être un bon compromis de ce point de vue.

En pratique, ce phénomène néfaste est un peu atténué du fait que le corps 2 n'est pas placé au contact direct du tube à rayons X d'une part et du multidétecteur d'autre part. Il occupe une position intermédiaire. Sur la figure 6b, la position intermédiaire du corps est matérialisée entre les droites 41 et 42 tandis que des trapèzes d'exploration respectivement GHIJ et KLMN sont présentés. Ces trapèzes tête bêche correspondent à des surfaces de tranche irradiée quand le tube X se trouve dans une position donnée (position de la figure 1 par exemple) et dans une position symétrique (par rapport à l'axe 12) de cette position donnée. On remarque que des régions de l'objet présentées avec des petites croix sont irradiées pour contribuer à une vue selon une orientation donnée alors qu'elles ne sont pas irradiées pour contribuer à une vue symétrique dans son orientation de la vue en question.

Dans un premier temps on peut ignorer ce type de défaut théorique et reconstruire les images comme si le rayonnement était parfaitement de type parallèle. Par ailleurs, on peut envisager un mode de fonctionnement ou la source X 2 ne subit pas de mouvement de translation parallèle à l'axe 12 mais seulement un mouvement de rotation. Dans ce but le détecteur par son mouvement de translation fournit à chaque position angulaire les données correspondant à une projection conique de l'objet. On peut montrer qu'un ensemble de projections coniques, correspondant à un mouvement circulaire de la source, ne permet pas en toute rigueur de reconstruire l'objet. Il faut un mouvement plus complexe de la source. Toutefois un mouvement circulaire permet une reconstruction approchée. Des algorithmes de reconstruction à partir de projections coniques sont disponibles. Ils sont complexes et demandent une grande puissance de calcul, mais sous une forme simplifiée dans un cas où la source ne décrit qu'un mouvement circulaire, l'invention est facilement applicable.

## Revendications

1 - Dispositif de tomographie comportant
- un ensemble (1) tube (2) à rayons X-multidétecteur (4) muni d'un tube à rayons X agencé pour rayonner en direction (3) d'un multidétecteur avec lequel il est mécaniquement (5,6) associé,
- des moyens (60) de rotation pour faire tourner cet ensemble autour d'un axe (12) de rotation passant transversalement entre le tube à rayons X et le multidétecteur, cette rotation correspondant à un angle minimum d'exploration nécessaire à une reconstruction d'un objet interposé entre le tube et le multidétecteur,
- des moyens (7,8,50) de translation pour déplacer au moins le multidétecteur le long de cet axe de rotation, la longueur de cette translation correspondant à la longueur d'une région à examiner de l'objet,
- et des moyens (10) de traiter des signaux délivrés par le multidétecteur, ces signaux résultant d'émissions du tube à rayons X, en vue de produire des images de l'objet (16) interposé, caractérisé en ce qu'il comporte en outre
- des moyens pour faire tourner l'ensemble d'un angle égal à cet angle minimum, de telle façon qu'une vitesse moyenne tangentielle du multidétecteur soit moins rapide que sa vitesse de translation et donc que cette rotaton se produise pendant une durée totale plus longue que la durée nécessaire pour que cet ensemble ne se déplace en translation au moins et fois le long de toute cette longueur à examiner,
- et des moyens pour faire émettre le tube à rayons X au cours du déplacement en translation.

2 - Dispositif de tomographie comportant
- un ensemble (1) tube (2) à rayons X multidétecteur (4) muni d'un tube à rayons X agencé pour rayonner en direction (3) d'un multidétecteur avec lequel il est mécaniquement (5,6) associé,
- des moyens (60) de rotation pour faire tourner cet ensemble autour d'un axe (12) de rotation passant transversalement entre le tube à rayons X et le multidétecteur,
- des moyens (10) de traiter des signaux délivrés par le multidécteur, ces signaux résultant d'émissions du tube à rayons X, en vue de produire des images d'objets (16) interposés ente ce multidétecteur et ce tube à rayons X,
- les moyens de rotation comportant des moyens pour faire tourner l'ensemble pas à pas (28), caractérisé en ce que
- les moyens de translation comportent des moyens pour déplacer (29-30), à chaque pas de rotation, l'ensemble en translation le long de toute une région à examiner des objets,
- et des moyens pour faire émettre le tube à rayons X au cours de chaque déplacement en translation.

3 - Dispositif selon la revendication 1 ou la revendication 2 caractérisé en ce que
- le multidétecteur est du type multirangées (Fig 2, 19-23) et,
- les moyens de traitement comportent des moyens (25) pour intégrer dans le temps des signaux délivrés par ce multidétecteur multirangées au cours du déplacement en translation de l'ensemble.

4 - Dispositif selon la revendication 1 à 3 caractérisé en ce que le tube et le multidétecteur sont contenus dans un capotage (92,94) maintenu fixe pendant la translation d'une part, et déplacé pendant les rotations d'autre part.

5 - Dispositif selon l'une quelconque des revendications 1 à 4 caractérisé en ce que les moyens de translation comportent des moyens pour déplacer l'ensemble dans un sens (29) puis dans un autre (30) d'une rotation (28) à une autre.

6 - Dispositif selon l'une quelconque des revendications 1 à 5 caractérisé en ce que les moyens d'associer le tube au multidétecteur ont une forme générale d'arc (6) de cercle ouvert.

7 - Dispositif selon l'une quelconque des revendications 1 à 6 caractérisé en ce que le multidétecteur est multirangées et est du type à dérive d'ions.

8 - Dispositif selon l'une quelconque des revendications 1 à 6 caractérisé en ce que le multidétecteur est multirangées et est du type à réseau de cellules détectrices à deux dimensions.

9 - Dispositif selon la revendication 8 caractérisé en ce que les cellules comportent des dispositifs à tranfert de charge.

10 - Dispositif selon la revendication 9 caractérisé en ce que le multidétecteur multirangées comprend un réseau d'éléments photodétecteurs.

11 - Procédé de tomographie comportant les étapes suivantes
- on fait tourner, d'un angle minimum d'exploration nécessaire à la reconstruction d'un objet, un ensemble (1) tube (2) à rayons X multi-détecteur (4), muni d'un tube à rayons X agencé pour rayonner en direction (3) d'un multidétecteur avec lequel il est mécaniquement (5,6) associé autour d'un axe (12) de rotation passant transversalement entre le tube à rayons X et le multi-détecteur,
- on déplace (29-30) l'ensemble en translation le long de toute une région à examiner des objets,
- et on traite (10) des signaux délivrés par le multi-détecteur, ces signaux résultant d'émissions du tube à rayons X, en vue de produire des images d'objets (16) interposés entre le multi-détecteur et le tube à rayons X, caractérisé en ce que
- on fait tourner l'ensemble d'un angle égal à cet angle minimum, de telle façon que le multidétecteur soit entrainé à une vitesse moyenne tangentielle moins rapide que sa vitesse de translation et donc que cette rotation se produise pendant une durée totale plus longue que la durée nécessaire pour que cet ensemble ne se déplace en translation au moins une fois le long de toute cette longueur à examiner.

12 - Procédé selon la revendication 11 caractérisé en ce que
- on fait émettre le tube à rayons X au cours de chaque déplacement en translation.

FIG_1

FIG_2

FIG_3-a

FIG_3-b

FIG_4-a

FIG_4-b

FIG_4-c

DEBUT

Acquisition d'une vue

V$_1$

V$_2$

Vn

V$_n$

TRANSLATION

Rotation pas à pas

Retroprojection

R$_1$

R$_2$

Rn

RETROPROJECTION

I$_1$

I$_2$

In

FIN

FIG_5

FIG_6-a

FIG_6-b

32  33  34  35  36

31  37  38  39  40

G  M  H  42

41

K  J  I  N

# FIG_7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 063 097  (D.M. BARETT et al.) <br> * Résumé; colonne 2, lignes 13-40; colonne 5, lignes 41-61; colonne 7, lignes 50-65; colonne 8, lignes 43-68; colonne 16, lignes 17-47; colonne 17, ligne 7 - colonne 18, ligne 10; figures 1,2,20,23 * | 1,2,4, 11 | A 61 B   6/03 |
| A | --- | 5-7,9 | |
| Y | EP-A-0 099 018  (SIEMENS AG) <br> * Résumé; page 2, lignes 9-31; page 3, ligne 16 - page 4, ligne 15; page 6, lignes 1-27; page 7, lignes 17-21; figures 1,3,6 * | 1,2,4, 11 | |
| A | EP-A-0 024 028  (SIEMENS AG) <br> * Résumé; page 2, ligne 32 - page 3, ligne 10; page 3, ligne 22 - page 4, ligne 21; page 6, lignes 1-32; figures 1-3 * | 1-3,6,8 | |
| A,D | EP-A-0 051 350  (TECHNICARE CORP.) <br> * Résumé; page 7, lignes 7-12; page 8, lignes 6-35; page 9, lignes 30-36; page 10, ligne 19 - page 11, ligne 4; figures 1,2 * | 1-3,6,8 -10 | **DOMAINES TECHNIQUES RECHERCHES (Int. Cl.4)** <br><br> A 61 B |

Le présent rapport a été établi pour toutes les revendications

| Lieu de la recherche | Date d'achèvement de la recherche | Examinateur |
|---|---|---|
| LA HAYE | 07-08-1989 | RIEB K.D. |

EPO FORM 1503 03.82 (P0402)